# EUROPEAN PATENT SPECIFICATION

(11) **EP 3 226 788 B1**
(45) Date of publication and mention of the grant of the patent: **29.01.2020**
(21) Application number: 15816375.8
(22) Date of filing: 30.11.2015
(51) Int. Cl.: A61B 17/64, A61B 17/86

(54) **ORTHOPEDIC EXTERNAL FIXATION SYSTEM FOR THE TREATMENT OF COMPLEX FRACTURES UNDER PRECARIOUS SURGICAL ENVIRONMENTAL CONDITIONS**
ORTHOPÄDISCHES EXTERNES FIXATIONSSYSTEM ZUR BEHANDLUNG VON KOMPLEXEN FRAKTUREN UNTER PREKÄREN CHIRURGISCHEN UMGEBUNGSBEDINGUNGEN
SYSTÈME DE FIXATION ORTHOPÉDIQUE EXTERNE POUR LE TRAITEMENT DE FRACTURES COMPLEXES DANS DES CONDITIONS D'ENVIRONNEMENT CHIRURGICAL PRÉCAIRES

(30) Priority: 04.12.2014 EP 14196301
(43) Date of publication of application: 11.10.2017
(73) Proprietor: Orthofix S.r.l., 37012 Bussolengo (VR) (IT)
(72) Inventor: VENTURINI, Daniele, 37064 Povegliano Veronese (Verona) (IT); MARINI, Graziano, 37060 Castel d'Azzano (Verona) (IT)
(74) Representative: Botti, Mario
(86) International application number: PCT/EP2015/078020
(87) International publication number: WO 2016/087347

(56) References cited:
- EP-A2- 2 774 560
- US-A1- 2005 065 524
- US-A1- 2009 118 733
- US-A1- 2011 130 794

## Description

### Field of application

The present invention relates to an integrated orthopedic external fixation system for the treatment of complex or displaced fractures under precarious environmental conditions which often exist in third world countries, of the type comprising an external fixator and a plurality of bone screws for fixing the fixator to a fractured long bone.

More particularly, the invention relates to a parts kit for assembly of the aforementioned system and the description which follows will be provided with reference to this particular arrangement of component parts with the aim of simplifying the illustration thereof.

### Prior art

As is well known in this specific technical sector, the treatment of complex fractures, in particular exposed fractures, always poses a major problem for an orthopedic doctor.

The problem is further complicated in all those cases where it is required to treat these injuries in precarious environmental conditions where the normal surgical equipment available at a clinic or in a suitably equipped operating theater is missing.

This is the case, for example, with traumatology as managed in developing or even underdeveloped countries where there are even hygiene-related problems.

In these countries the operating theater where surgical orthopedic devices are fitted normally do not have intraoperative examination equipment, pneumatic drills or an operating table suitable for reducing the fractures.

Also for the normal activities of the orthopedic surgeon, in connection with the external fixation systems of the prior art and, in particular, for the tightening of structural screws or articulations or clamps and the associated jaws, the lack of suitable spanners or sterilized material may constitute a problem. Often insertion of the screws poses a major problem owing to the lack of suitable or biocompatible drills or also the lack of screw guides or hole guides.

The drills used are even of commercial origin and not designed specifically for surgical use.

Moreover, normally there does not exist the possibility of performing automated boring of the medullary canal and sterilization of the surgical equipment and the operating material is performed using steam.

In these types of environment the radical design of the devices and simplification of the technology are of the fundamental importance for the success of the implant.

At present, in the aforementioned precarious environmental conditions, the following various orthopedic surgery devices, suitable for the different applications depending on the anatomical site, are used:
Endosseous or medullary pins are applied for the treatment of fractures of the trochanter area of the femur and for diaphyseal fractures of the femur and tibia, while plates are used in the vicinity of the main articulation zones (knee, tibial pilon and elbow).

External fixators are used for exposed and tibial fractures.

For example, an integrated orthopedic external fixation system suitable for treatment of complex or displaced fractures under precarious enviromental conditions is disclosed in the US patent application nr. US 2009/0118733.

This document relates to an orthopedic device for fixating bone parts includes a set of one or more bone fixation pins for engaging a bone piece, an elongated fixator body having an internally threaded bore extending longitudinally therethrough, a pair of opposed longitudinally extending slots in the fixator body, and a pin-holder received within the fixator body and configured to removably hold the set of one or more bone fixation pins. The pin-holder is longitudinally movable within the fixator body for controlling the position of the set of bone fixation pins within the fixator body. The fixator body also can be configured to removably hold a second set of one or more bone fixation pins at a location fixed along the fixator body. However, this solution is not so easily adjustable in environments wherein it does not exist the possibility of performing automated boring of the medullary canal and sterilization of the surgical equipment.

In those precarious environmental conditions screws or wires are used for stabilization of the smaller bones.

The technical problem underlying the present invention is that of devising an orthopedic external fixation system for the treatment of complex or displaced fractures under precarious environmental conditions, namely conditions where there is limited hygiene and/or accessory equipment available for assembly of the component parts of the orthopedic system, these conditions occurring in many underdeveloped countries.

Another object of the present invention is to devise an integrated orthopedic system having structural and functional characteristics such as to allow the use thereof in surgical environments which however do not have the normal equipment available in the operating theaters of advanced countries.

A further object of the present invention is to provide an orthopedic external fixation system capable of ensuring adequate stability for the entire duration of the treatment until complete healing of the fracture occurs, thus also allowing the patient to apply a load thereon early on.

### Summary of the invention

The aforementioned objects are achieved by an integrated orthopedic external fixation system of the type mentioned above and characterized in that it comprises:
- a pair of cylindrical portions interconnected coaxially together by means of interlocking connection means able to adjust the movement towards and away from each other of these portions;
- a plurality of through-holes formed transversely with respect to the axis of the body of the fixator and at a predefined distance from the opposite free ends of the fixator, for receiving a respective bone screw;
- each bone screw having a smooth cylindrical central section and being slidably engaged in a guided manner inside the corresponding hole of the body of the fixator;
- a threaded front tip portion, intended to come into screwing engagement with said long bone, in a substantially self-tapping manner; and
- a threaded rear tip portion intended to come into screwing engagement with the corresponding hole of the fixator body, in a substantially self-tapping manner.

Advantageously, each of said cylindrical portions is formed with a relatively soft, but not yielding material, for example a plastic synthetic material or a natural material or a metal alloy.

The portions of the cylindrical body of the fixator are coaxially and slidably connected together by means of a male/female joint between the end of one portion and a corresponding end seat of the other portion and in that this configuration is locked by means of tightening of a pair of through screw-bolts provided with associated nuts; one of said screw-bolts passing through a slot-shaped hole so as to allow a predefined axial sliding movement before tightening of the corresponding nut.

In apreferred embodiment the body of the fixator is cylindrical and made of solid material.

As an alternative, the body of the fixator may be tubular.

Moreover, one of said cylindrical portions is longer than the other and two components of the cylindrical body of the fixator are connected together by means of a male/female joint.

It shuold be further notd that the cylindrical body of the external fixator is provided with a plurality of through-holes distributed uniformly along a substantial section starting from each free end of the cylindrical components and structured to receive a relative bone screw.

Said through-holes are arranged and regularly spaced along at least three parallel lines and the center line of said three parallel lines has holes staggered with respect to the holes of the adjacent lines.

The body of the fixator presents two or more holes with a smaller diameter if compared to the diameter of said through-holes; those smaller holes are provided in the vicinity of the free ends of each of the two components of the fixator body for a preliminary stabilization of the fixator using K-wires or Kirschner wires.

The system according to the invention uses special bone screws characterized by having:
- a smooth cylindrical central section, which is slidably engaged in a guided manner inside a corresponding hole of the body of the fixator;
- a threaded front tip portion, intended to come into screwing engagement with said long bone, in a substantially self-tapping manner; and
- a threaded rear tail portion intended to come into screwing engagement with the corresponding hole of the fixator body in a substantially self-tapping manner.

The tip portion of the screw is self-perforating and self-tapping with an undercut groove.

The screw has a threaded end for bone engagement having a cylindrical shape, obtained by forming an helix on a stem of 5 mm diameter with pitch of 0.75 mm so as to allow a good grip also in a spongy bone.

The thread of the screw forms a double angle of 30° at the vertex and 150° on the bottom with a thread height of 0.5 mm.

Further characteristic features and advantages of the orthopedic system according to the invention will emerge from the description, provided hereinbelow, of a non-limiting example of embodiment thereof with reference to the accompanying drawings.

### Brief description of the drawings

- Figure 1 shows a perspective view of an orthopedic external fixation system provided according to the present invention for the treatment of complex or displaced fractures;

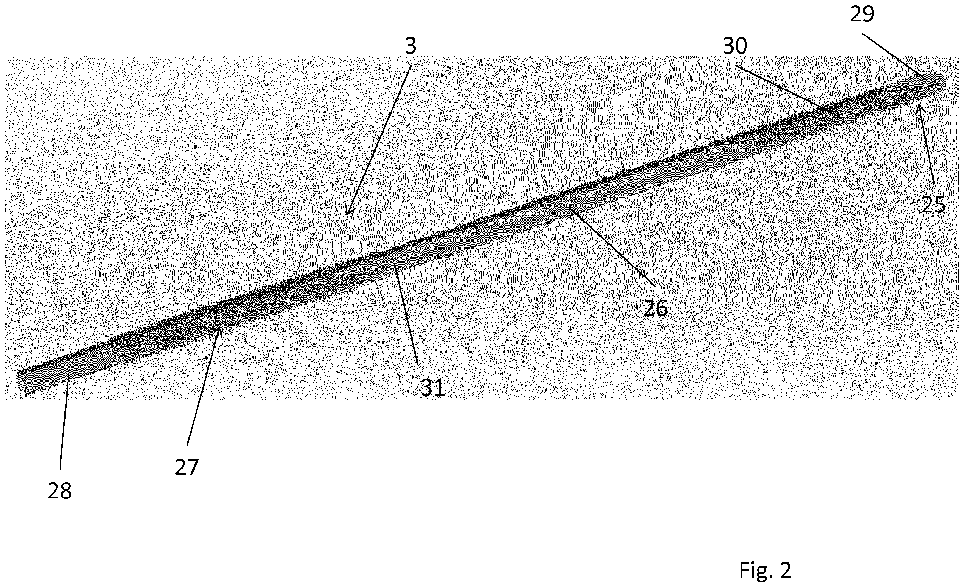

- Figure 2 shows a perspective view of a detail of a component of the system according to the invention;
- Figure 3 shows a perspective view of a detail of a component of the system according to the invention;

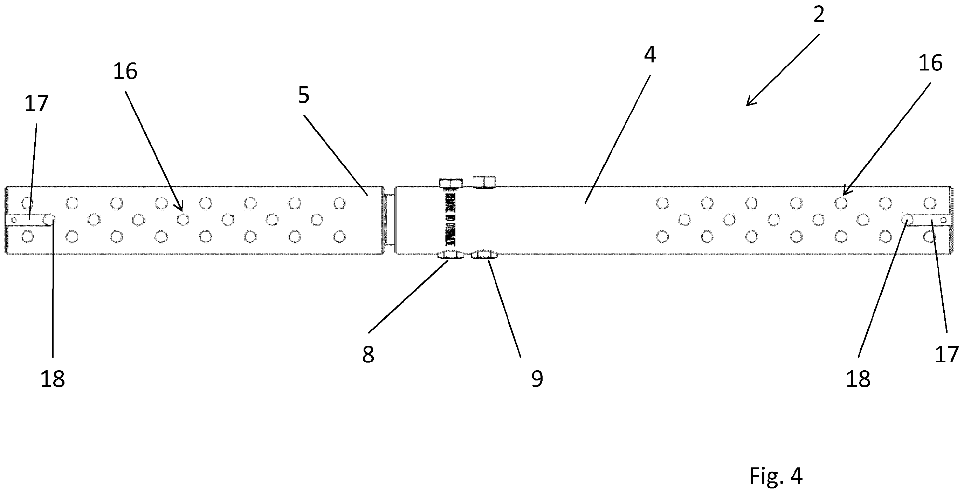

- Figure 4 shows a longitudinal side view of a detail of a component of the system according to the invention;
- Figure 5 shows a longitudinally sectioned view of a detail of the system according to the invention;

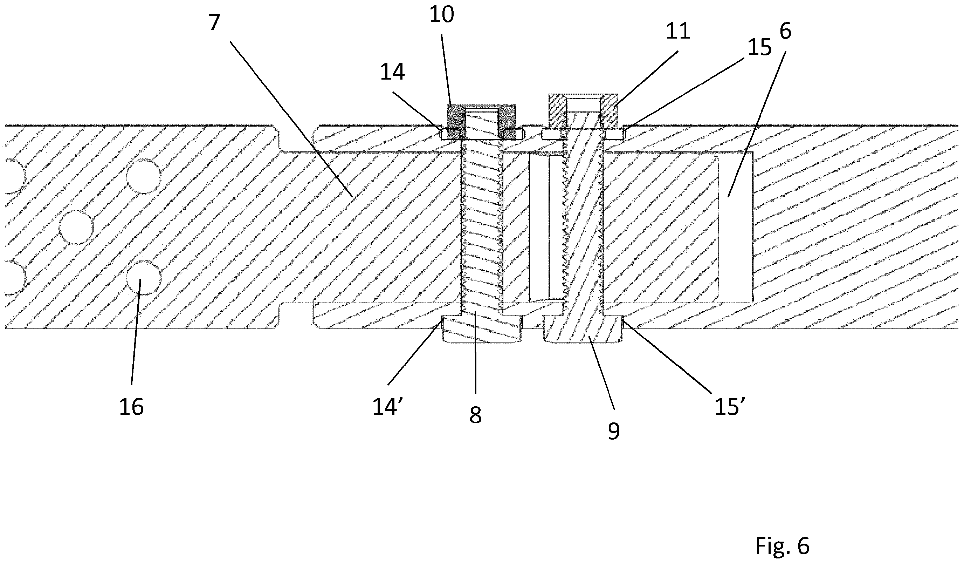

- Figure 6 shows a cross-sectional view, on a larger scale, of the detail of Figure 5;

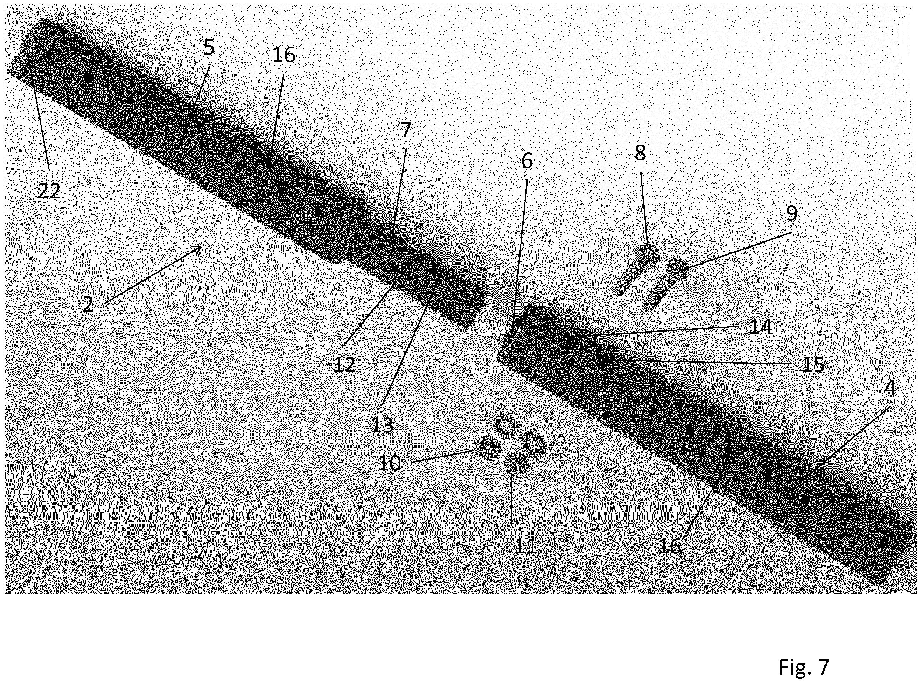

- Figure 7 shows an exploded perspective view of the component shown in Figures 3, 4, 5 and 6;
- Figure 8 shows a perspective view of the system according to the invention during a stage of assembly in situ on a femoral long bone;
- Figures 9 to 15 show respective perspective views of parts or overall views of the system according to the invention during different stages of assembly;
- Figure 16 shows a perspective view of a variation of embodiment of the detail shown in Figure 3;
- Figures 17 to 26 show respective perspective views of details or overall views of a variation of embodiment of the external fixation system according to the invention during different stages of assembly in situ on a tibial long bone.

### Detailed description

With reference to these figures, 1 denotes overall and in schematic form an integrated orthopedic system realized according to the present invention for external fixation and the treatment of complex or displaced fractures under precarious environmental conditions.

"Precarious environmental conditions" is understood as meaning environments where all the normal surgical equipment available at a clinic or in a suitably equipped operating theater is missing. More particularly, it refers to the manner in which surgical orthopedic traumatology is managed in underdeveloped countries where even hygiene-related problems exist and where sterilization of the surgical instruments or components of the orthopedic fixators is performed using steam.

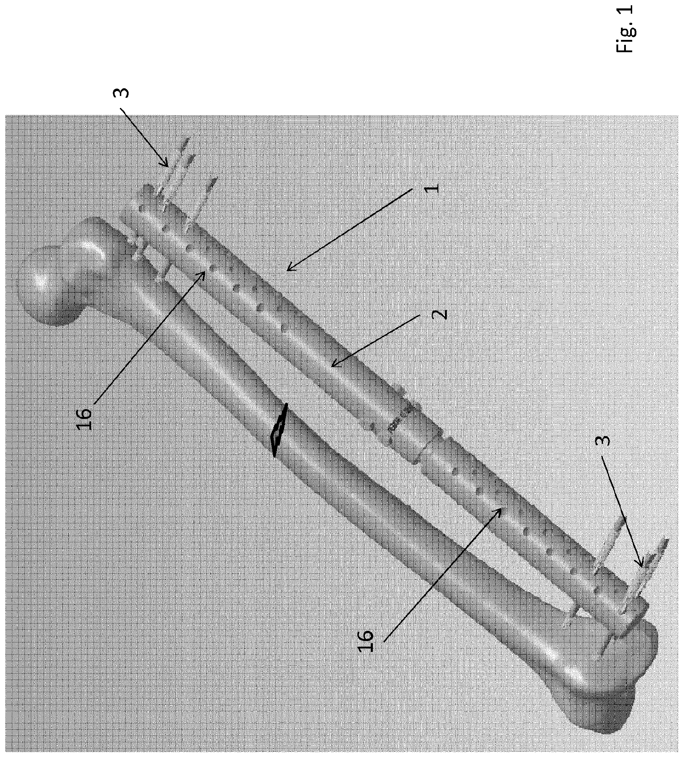

The system 1 comprises an external fixator 2 and a plurality of bone screws 3 for connecting the fixator 2 to a fractured bone, as shown in Figure 1. The bone screws pass through holes 16 formed transversely with respect to the longitudinal axis X of the fixator 2.

The external fixator 2 is an elongated cylindrical body. In a preferred embodiment this cylindrical body is made of solid material, but it is also possible, depending on the material forming it, for the body 2 to be made with a tubular form.

Preferably, the external fixator 2 comprises at least a pair of components 4 and 5 which are fastened together. It is possible, however, for the cylindrical body of the fixator 2 to consist of one piece.

Advantageously, the two components 4 and 5 of the cylindrical body 2 are in turn formed so that they can be connected together by means of a quick-fitting or interlocking system. One 4 of the two components of the cylindrical body 2 is overall longer than the other one, but in this case also it is possible for the two components to have the same length.

In any case, the longer component 4 has a cylindrical seat 6 inset in one of its ends. The other, shorter, component 5 instead has an end 7 with a cylindrical shape, but a diameter smaller than that of its main circular cross-section and substantially corresponding to the internal diameter of the seat 6.

The seat 6 is relatively deep, so that it occupies nearly a quarter of the entire length of the component 4.

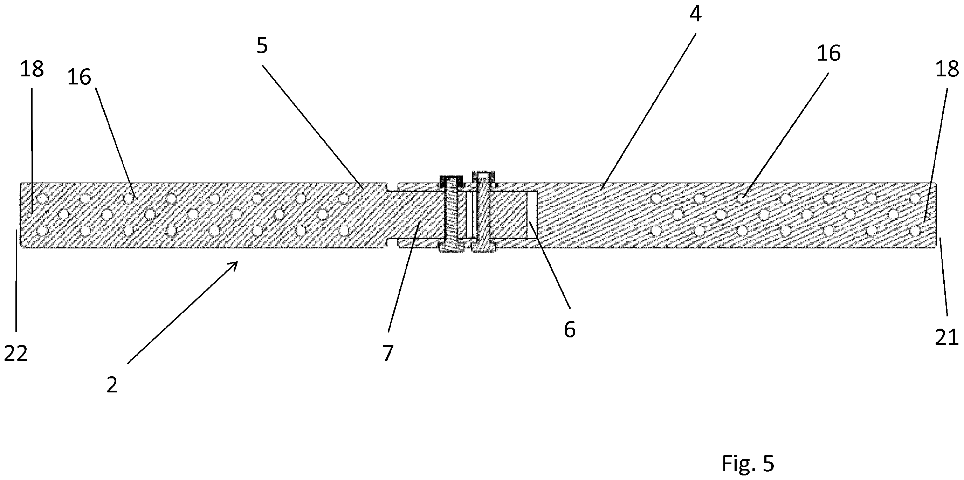

The two components 4 and 5 of the cylindrical body of the fixator 2 are connected together by means of a male/female joint between the end 7 of the component 5 and the seat 6 of the component 4 so as to form the longitudinally extending cylindrical body 2, as clearly shown in the cross-sectional view of Figure 5.

This configuration, which is essentially of a sliding nature, is locked by tightening a pair of through screw-bolts 8, 9 provided with associated nuts 10, 11.

The screw-bolts 8 and 9 pass through respective oppositely arranged holes 14, 14' and 15, 15' formed in the end of the cylindrical component 4 in which the seat 6 is formed.

Similarly, the screw-bolts 8 and 9 pass through respective holes 12 and 13 formed in the end 7 of the cylindrical component 5.

The holes 12 and 13 are coaxial with the corresponding holes 14, 14' and 15, 15' so that when the ends 6 and 7 are connected together, the holes are also aligned with each other.

It should be noted, however, that the hole 13 in the end 7 is elliptical or slot-shaped so as to allow a predefined axial sliding movement before the corresponding nut 11 is tightened. The purpose of this particular configuration will become clear from the description below.

It should also be noted that the cylindrical body of the external fixator 2 is provided with a plurality of through-holes 16 distributed uniformly along a substantial section, i.e. at least half of the entire extension of the fixator, starting from each free end 21 or 22 of the cylindrical components 4 and 5.

The holes 16 have a diameter of about 5 mm and are arranged and regularly spaced along at least three parallel lines at a distance of about 7.5 mm from each other. The center line has holes 16 staggered by about 10 mm with respect to the holes of the adjacent lines; while the interval between the holes 16 along the center line is about 20 mm.

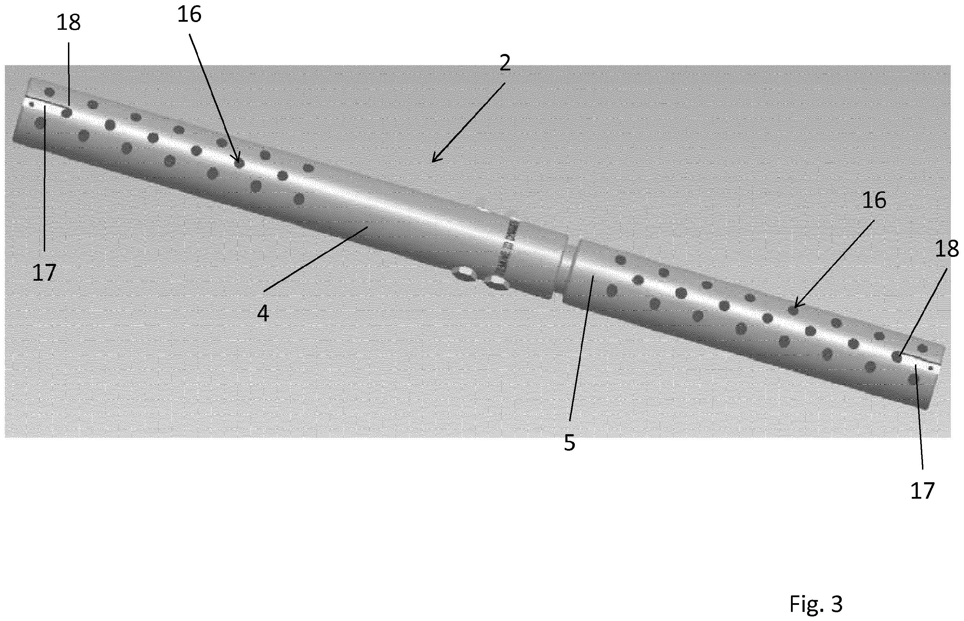

The holes 16 are in any case aligned with each other in parallel rows. It may also be considered that the holes of one row are staggered with respect to the holes of an adjacent row to as to resemble the vertices of rhombuses, as can be clearly seen in Figure 3.

The holes 16 are intended to be passed through by the bone screws 3.

The fixator 2 has an essential and basic structure; in fact it can be clearly noted that it has an unusual structural simplicity and is entirely devoid of articulations and clamps and associated jaws. The fixator 2 consists of a cylinder of relatively soft, but not yielding material, for example a plastic synthetic material which may be obtained from a bar or a rapid prototyping process; however, a natural material such as wood may be equally well used or found locally, or also a metal alloy, such as an aluminum alloy. The dimensions of the diameter are equal to about 30 mm for a length of about 400 mm. The body of the fixator 2 ensures positioning stability and flexibility of the screws 3 in the various anatomical sites and with different configurations of the screws 3 in order to ensure stability.

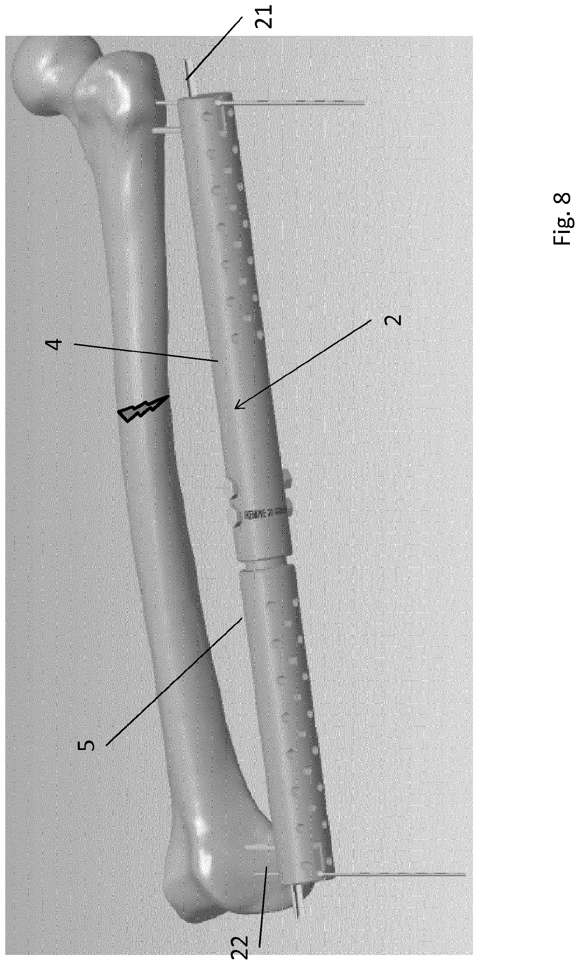

Two or more holes 18 with a smaller diameter, compared to the through-holes 16, are provided in the vicinity of the free ends of each of the two components 4, 5 of the fixator 2. These holes 18 are also through-holes 18 and are provided for preliminary stabilization using K-wire or Kirschner wire, with a diameter of 2.2 mm, as shown in Figure 8.

These holes 18 are formed at the end of a respective groove 17 present at each free end of the fixator. Opposite grooves 17 are provided on opposite sides of each free end 21 or 22.

The fixator 2 is also structured to allow the possibility of dynamic operation by means of axial sliding of the components 4 and 5, enabling axial compressive sliding over a distance of at least 5 mm by removal of the nut 11 which tightens the screw-bolt 9 inside the elliptical hole 13.

The second nut 10 has the function of maintaining the torsional stability, allowing axial travel during the dynamic operation step.

The penetration of the end 7 inside the corresponding seat 6 along about a quarter of the length of the associated portion 4 of the fixator body allows the formation of a telescopic stem connection which ensures axial sliding without any jamming during the movement.

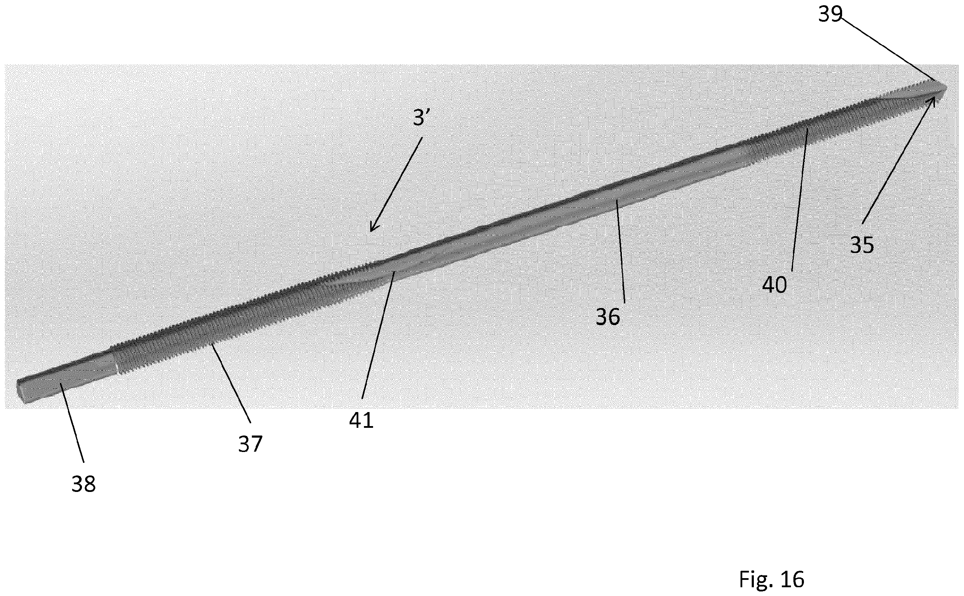

Now, with particular reference to the examples shown in Figures 3 and 16, another fundamental component of the system 1, namely one of the bone screws 3, will be described in detail.

The principle on which stabilization of the system 1 according to the present invention is based is that of generating during insertion of the screw 3 into the bone automatic stabilization of the structure of said fixator body 2.

All this is possible owing to masking of a part of the thread of the bone screw 3 in the region of the holes 16 for receiving the bone screws 3 on the fixator body 2 and correct calibration of said holes 16 depending on the strength characteristics of the material of said fixator.

The direction and orientation of the holes 16 for stabilization of the bone screws 3 on the fixator 2 is in fact based on the anatomy and the access ways for stabilization of the various anatomical parts. It has already been mentioned that the through-holes 16 are transverse to the longitudinal axis X of the fixator 2, but the term "transverse" does not necessarily indicate a perpendicular direction.

The geometrical form of the bone screws 3 and the corresponding holes 16 on the fixator 2 are calibrated depending on the anatomical site to be stabilized.

The femoral bone screw has the following characteristic features:
- a threaded front or distal tip portion 25, intended to come into screwing engagement with said long bone, in a substantially self-tapping manner; and
- a central smooth portion 26;
- a threaded rear or proximal tail portion 27 intended to come into screwing engagement with the fixator body 2, 2', in a substantially self-tapping manner.

The screw diameter is appropriate for the size of the bone and the expected load; in the case in question a diameter of at least 5 mm and smooth (structural) stem.

The distal tip 25 is self-perforating and self-tapping with an undercut groove 29; this tip 25 is similar to the drilling bits of drills so as to allow easy penetration and engagement of the thread in the first cortex of the bone.

The thread 30 for bone engagement has a cylindrical shape, obtained by forming the helix on the 5 mm diameter with pitch of 0.75 mm (fine thread) so as to allow a good grip also in the spongy bone, avoiding prestressing with pressure the opposite cortex during the drilling approach step.

The length of the threaded section 30 for gripping the bone is equal to about 40 mm so as to ensure a bicortical grip in the femur, except in the distal part (femoral condyles) where the grip is with the first cortex and spongy bone, while when it is in the trochanter neck the grip is only in the region of the acetabulum.

The thread of the screw 3 forms a double angle of 30° at the vertex and 150° on the bottom with a thread height of 0.5 mm.

The central smooth part 26 has a free length of about 75 mm and is suitable for the soft femoral parts, ensuring suitable spacing between bone and grip point on the external fixator 2.

Advantageously the rear threaded tail 27 is provided for engagement with the fixator 2. The cylindrical thread of this rear tail portion 27 has an approximate length of 55 mm and pitch equivalent to the bone thread 30 (0.75 mm). This screw threaded tail 27 is formed by winding the helix around the 5 mm diameter and therefore projects with an outer diameter of 6 mm.

Four grooves 31 tangential to the section 27 of the rear threaded tail portion are provided so as to allow a correct self-tapping approach of the thread inside the hole 16 of the fixator.

A rear attachment tang 28 is also provided, said tang having a cylindrical part with a length of about 5 mm and a part with three flattened surfaces formed at 120° with a length of about 14 mm, so as to allow secure engagement on drill chucks with gripping jaws.

The two lateral grooves 17 have the function of guiding any screw guide tool which is intended to keep the fixator 2 in the correct position during insertion of the bone screws 3.

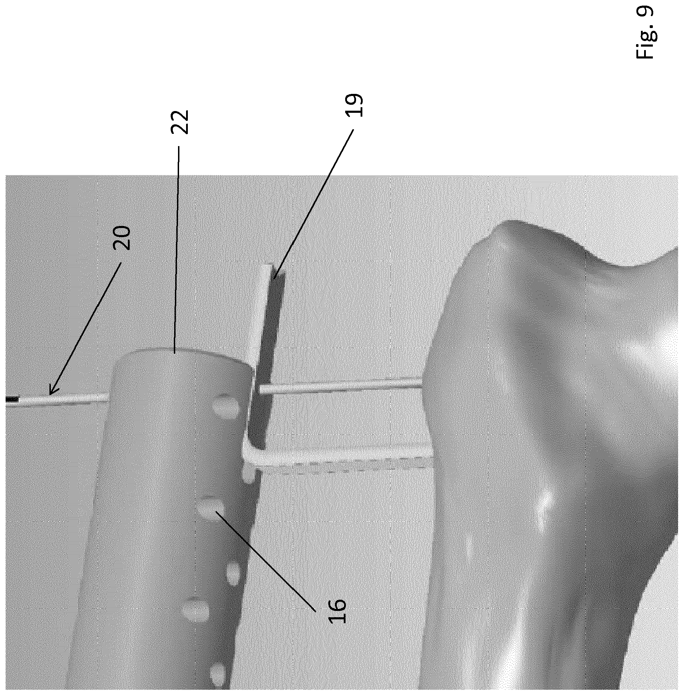

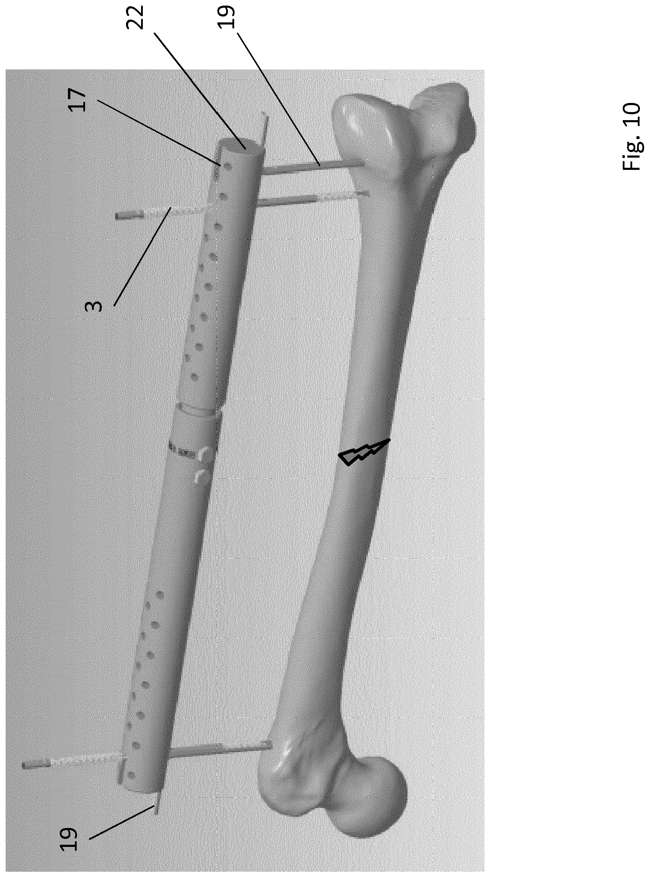

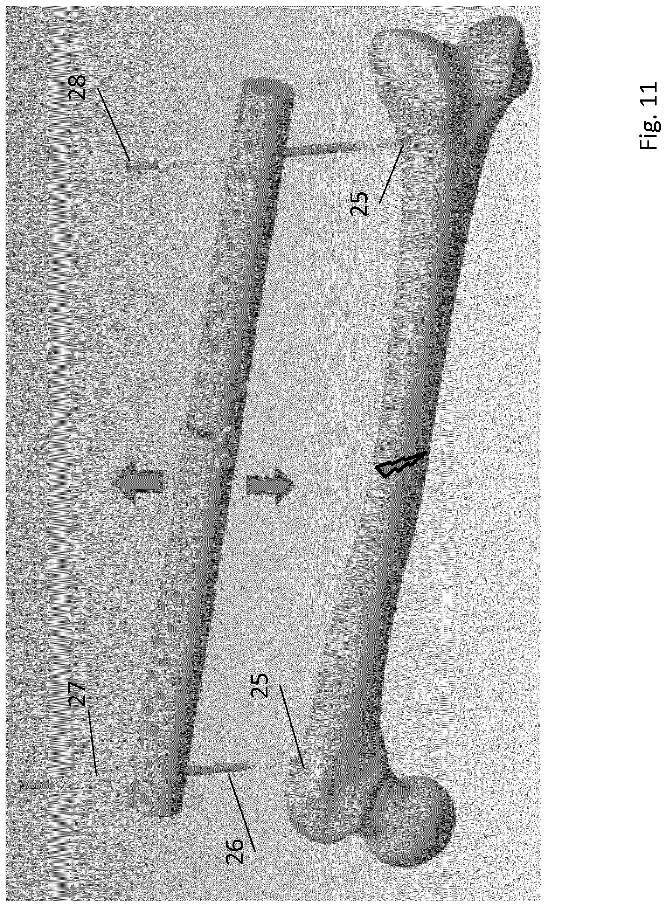

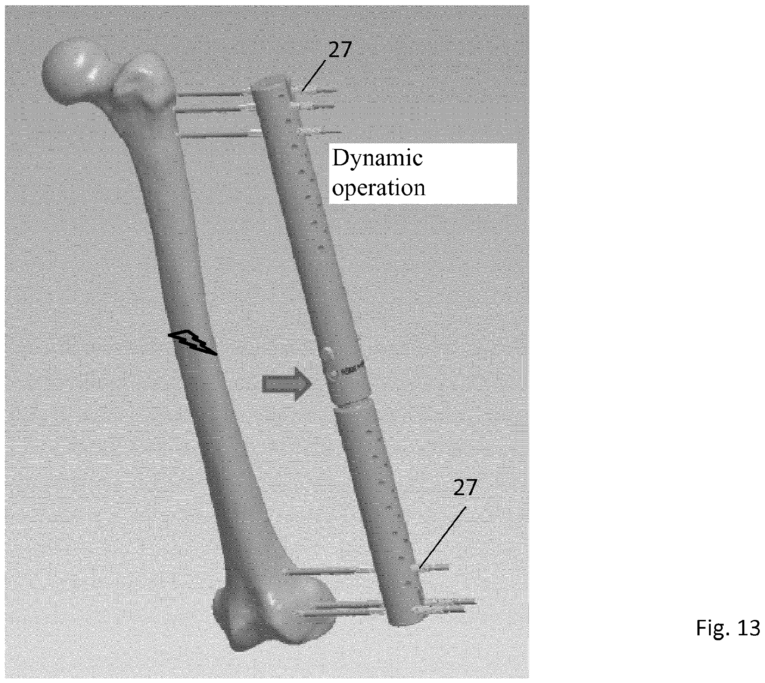

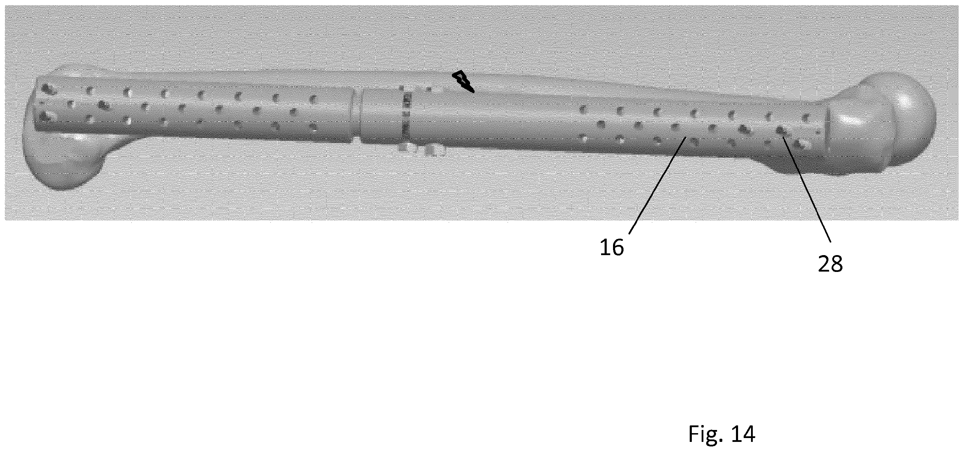

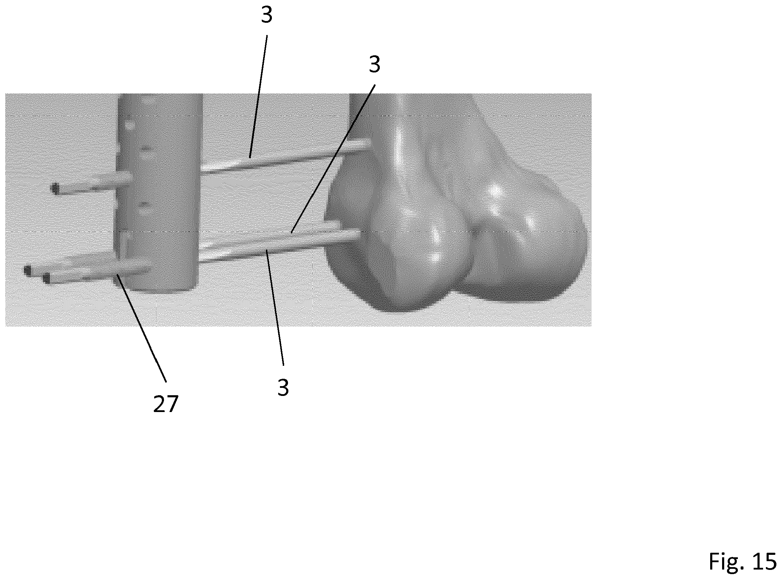

The pictures in Figures 8 to 15 illustrate a possible application of the system 1 on a femur and the flexibility of use of the engagement holes 16.

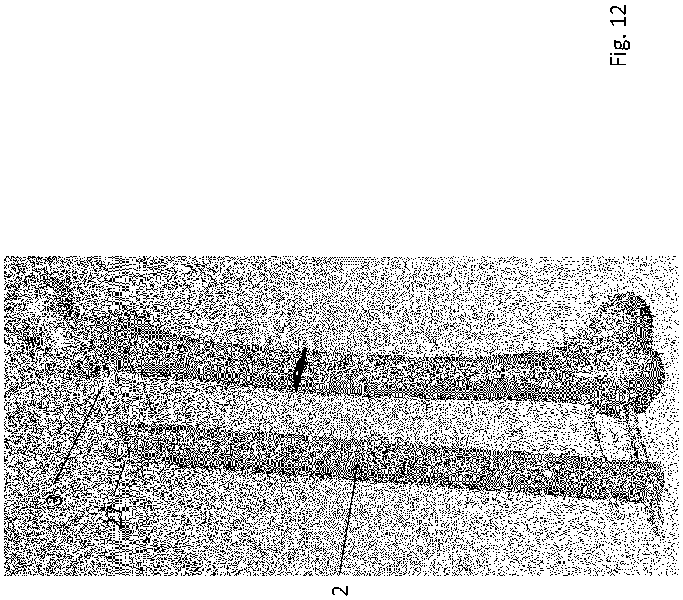

In the proximal position three screws 3 may be inserted, at least one being spaced or eccentric with respect to the axis X of the fixator 2 so as to follow the anatomical curvature of the femur, as shown for example in Figures 12, 13 and 14.

In the distal position instead, three screws may be applied in a triangular position, ensuring a high degree of stability in the various load planes and taking full advantage of the anatomical form of the femoral condyles.

We shall now consider briefly the operating technique for applying the fixing system according to the invention to the femur.

The system 1 according to present invention requires minimum tooling, which may be reproduced using basic technology at a minimum cost, for example by means of a lathe and a milling drill. All the components of the system 1 according to the invention may be steam-vaporized.

A few basic considerations should be made first for better understanding of the present invention.

The fracture must be reduced beforehand before the stabilization system 1 is applied. Insertion of the bone screws 3 may also be performed using a manual drill, although the procedure may be performed more quickly using an automated drill.

The technique involves firstly reduction of the fracture.

The body of the fixator 2 is arranged along the anatomical axis of the femur, the fixator is used as a template for receiving the thread 30 of the screw through a proximal hole 16 or a K-wire inside the corresponding hole 18 in the vicinity of one of the ends 21 or 22.

Alternatively, the tip 25 of the screw is screwed into the femur a few millimeters, checking that the fixator 2 does not engage with the stabilization threaded tail 27.

The more distal screw 3 is positioned and the tip 25 of the screw screwed in a few millimeters into the distal portion of the femur.

The distance of the fixator from the bone is set by sliding the fixator 2 along the smooth shank 26 of the screws 3.

A proximal screw 3 and a distal screw 3 are screwed into the bone until the desired depth is reached, checking also that the stabilization threaded tail 27 engages with the fixator. Then, it is possible to insert also the other proximal and/or distal screws 3 depending on the type and position of the fracture.

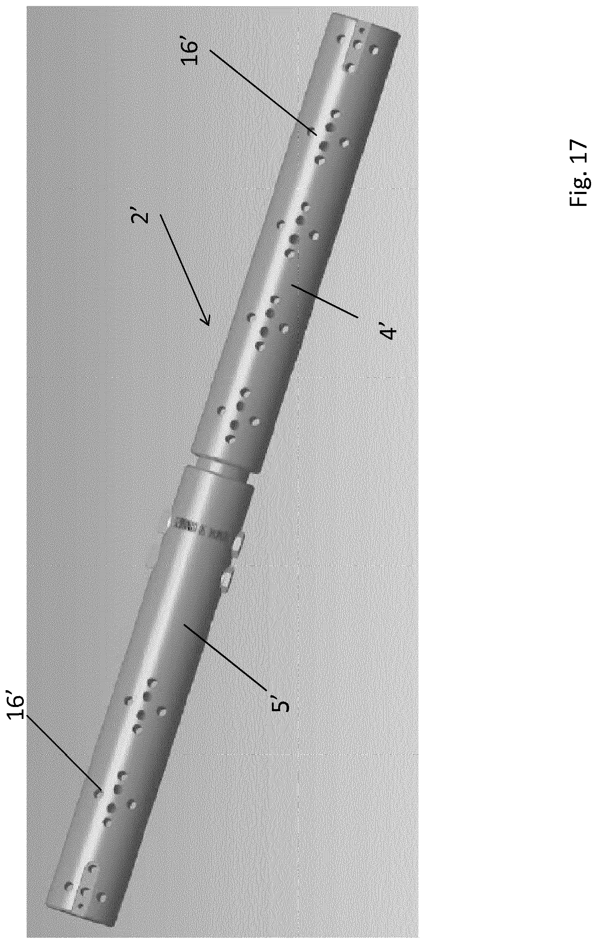

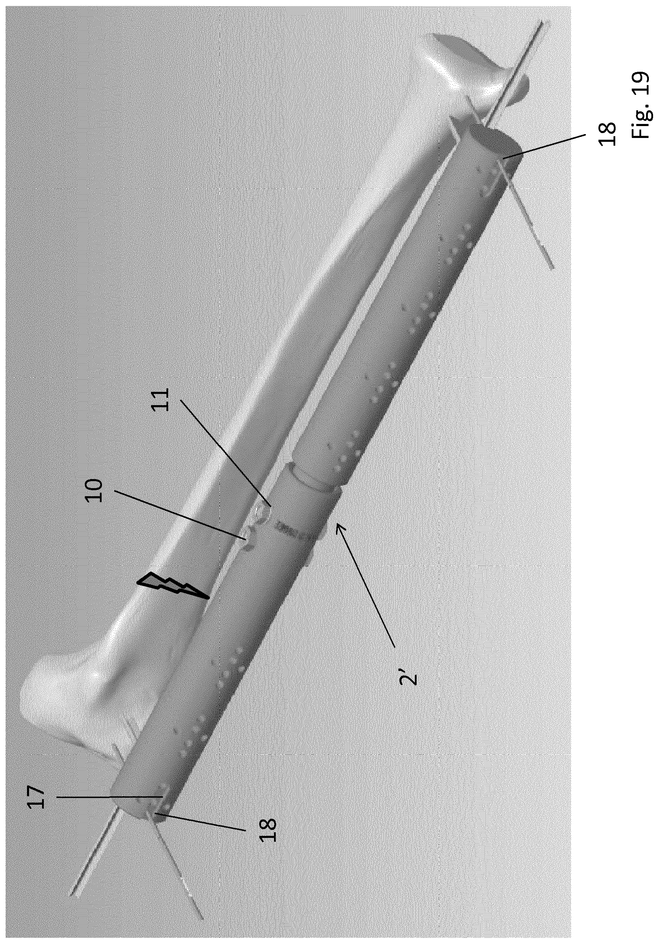

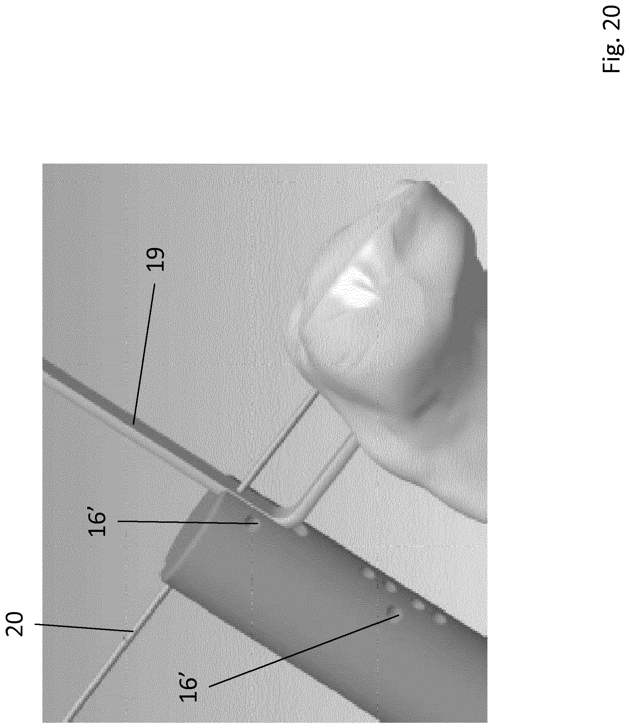

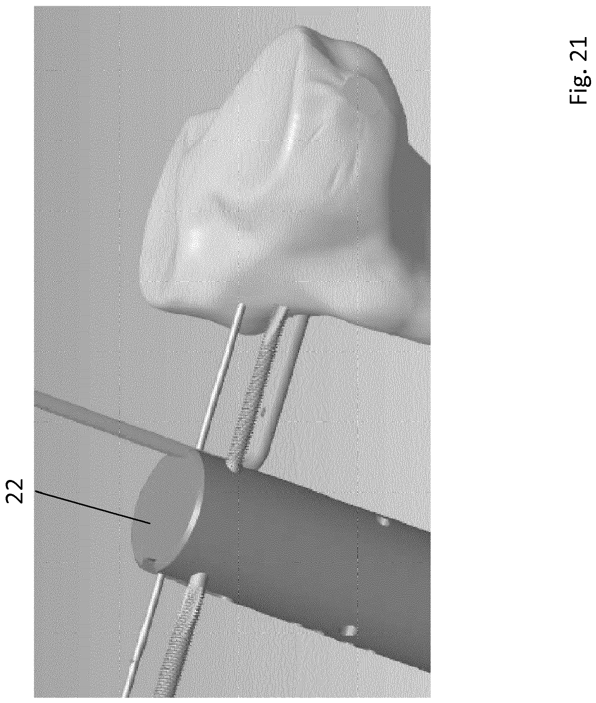

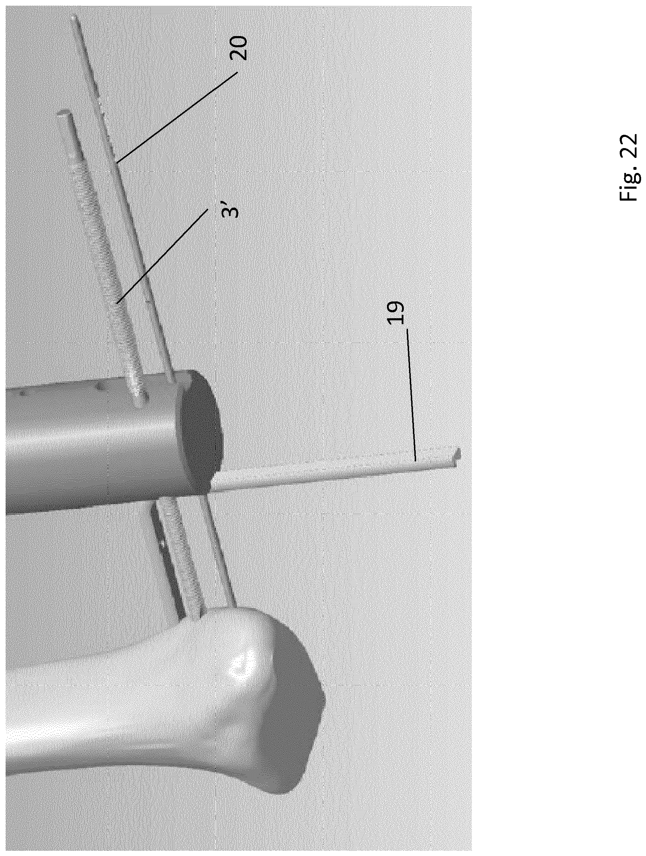

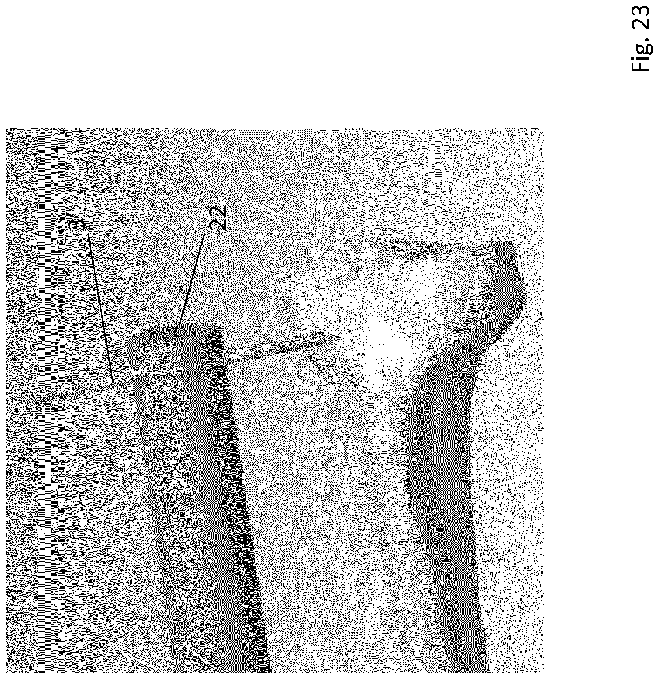

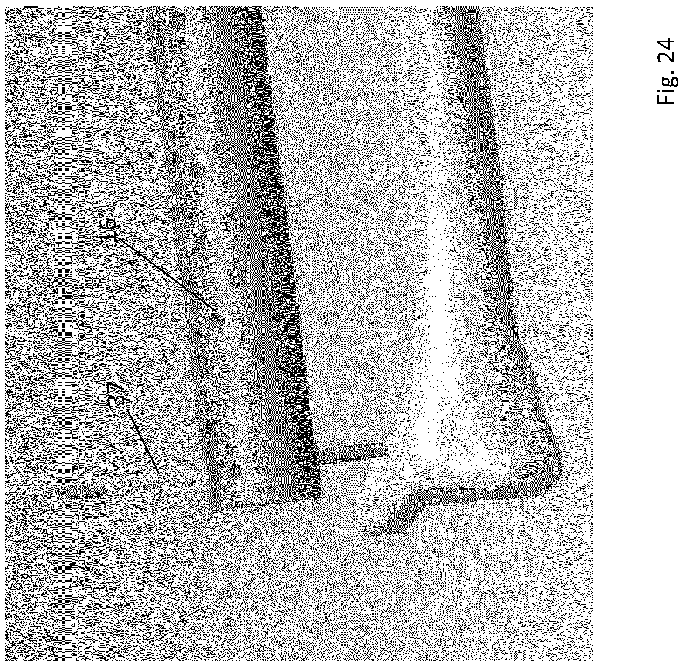

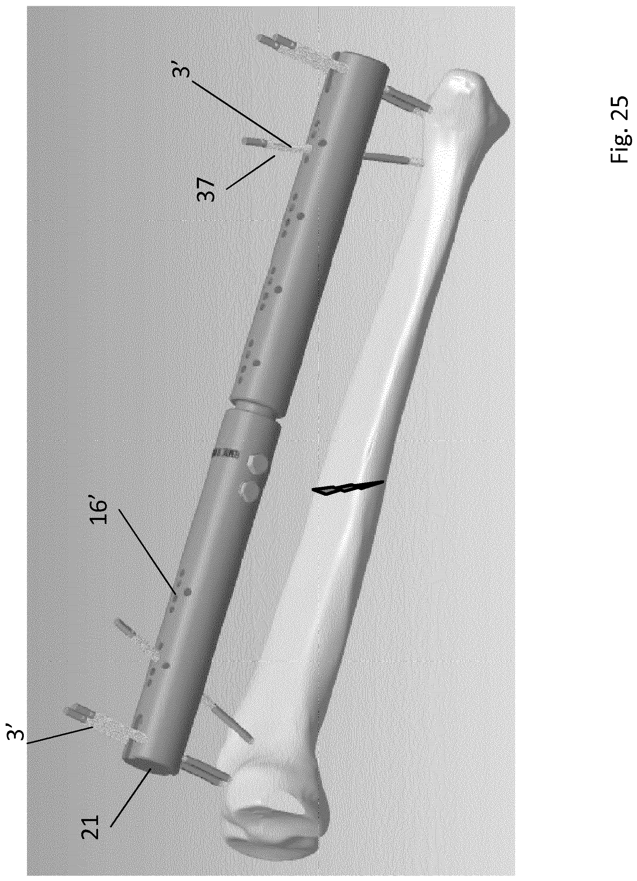

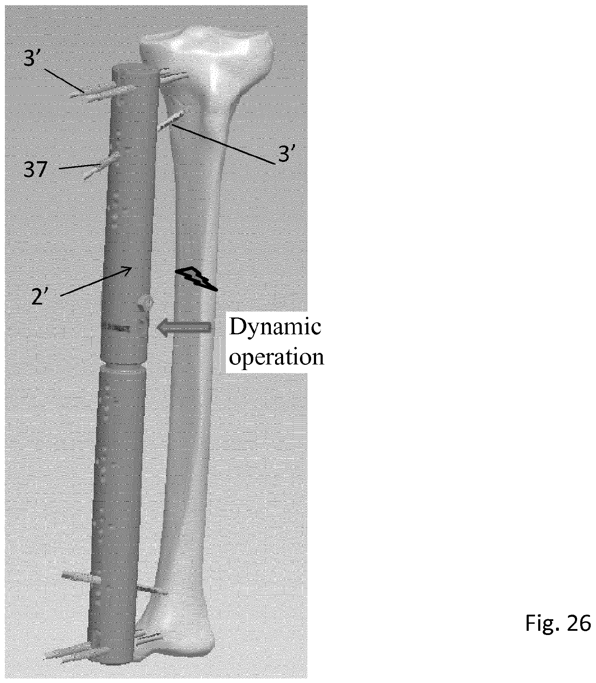

Figures 16 to 26 show examples of embodiment of a variant of the fixing system according to the present invention.

The body of the fixator is suitable for use in the specific areas of application to the femur or tibia. In the variant shown in Figures 16 to 26 we shall consider an example of embodiment relating to the tibia and shall indicate the body of the fixator by the number 2'.

Firstly it should be noted that the structure of the fixator 2' is substantially the same as the of the first embodiment with the sole difference consisting in the dimensions and the presence and distribution of the through-holes 16.

The fixator 2' has a simple structure and is composed of a solid cylinder of relatively soft, but not yielding material, for example a plastic synthetic material which may be obtained from a bar or a rapid prototyping process; however, a natural material such as wood may be equally well used and/or found locally, or also a metal alloy, such as an aluminum alloy. The dimensions of the diameter are about 30 mm for a length of about 380 mm; these measurements ensure the positioning stability and flexibility of the screws 3' in the various anatomical sites and with different configurations of the screws in order to ensure stability.

Two holes 18 for preliminary stabilization with K-wire of about 2.2 mm are present in the vicinity of the opposite free ends 21, 22.

Furthermore holes 16' with a diameter of about 4 mm are arranged along three parallel lines.

The central row has a series of holes which are perpendicular to the main axis X of the body 2' and spaced from each other by about 30 mm, being alternated with a pair of holes at about 30° relative to each other and spaced by 30 mm.

Laterally two rows of holes arranged at a distance of about 7.5 mm from each other are provided with a spacing of about 30 mm.

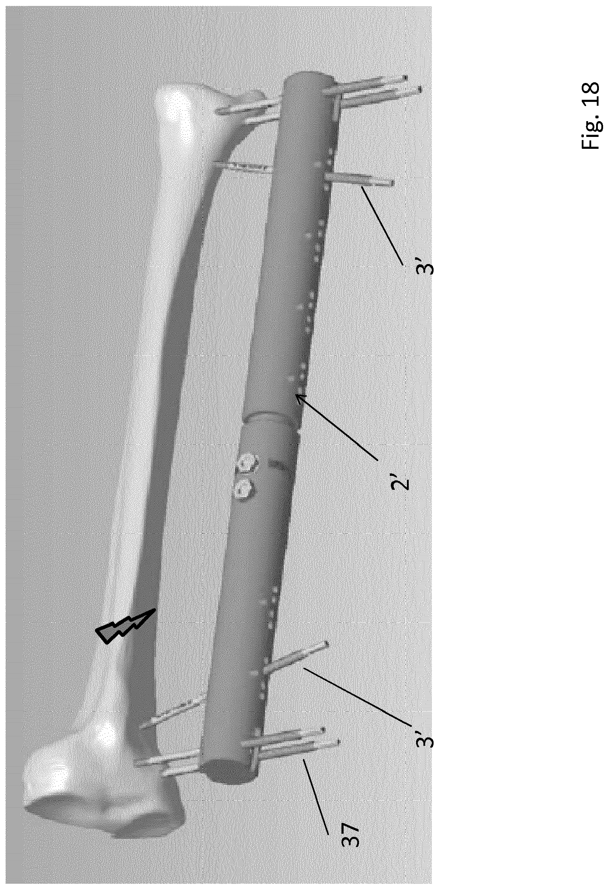

The pictures in Figures 18 and 24 to 26 illustrate a possible application on a tibia and the flexibility of use of the engagement holes 16'.

As regards the tibial bone screw 3' shown in Figure 16, it has an elongated form similar to that shown with reference to the femoral screw 3.

The diameter is appropriate for the size of the bone and the expected load; in particular a diameter of 4 mm with a smooth (structural) stem 36 was chosen.

The tip 35 is self-perforating and self-tapping with an undercut groove 39; the tip is similar to drilling bits so as to allow easy boring and engagement of the thread in the first cortex.

The thread 40 for bone engagement has a cylindrical shape, obtained by forming the helix on the 4 mm diameter with pitch of 0.75 mm (fine thread) so as to allow a good grip also in the spongy bone, avoiding prestressing with pressure the opposite cortex during the drilling approach step.

The length of the threaded section 40 for gripping the bone is about 40 mm so as to ensure a bicortical grip on the tibia, except in the proximal part (tibial plateau) where the grip is only with the first cortex and spongy bone.

The thread of the threading forms a double angle of 30° at the vertex and 150° on the bottom with a thread height of 0.5 mm.

The central smooth part 36 has a free length of about 30 mm; this part 36 is adapted to the necessary distances between the external fixator and the tibia.

A rear thread for engagement with the fixator 2 is provided. The threaded portion 37 is cylindrical with an approximate length of 62 mm and with a pitch equivalent to the bone screw (0.75 mm). The thread is formed by winding the helix around the 4 mm diameter and therefore projects with an outer diameter of 5 mm. Four grooves 41 tangential to the thread are provided so as to allow a correct self-tapping approach of the thread inside the hole of the fixator.

A rear attachment tang 38 has a cylindrical part with a length of about 3 mm and a part with three flattened surfaces formed at 120° with a length of about 10 mm, so as to allow secure engagement on drill chucks with gripping jaws.

We shall now consider briefly the operating technique for applying the fixing system according to the invention to the tibia.

The technique involves firstly reduction of the fracture.

The body of the fixator 2 is arranged along the anatomical axis of the tibia, the fixator is used as a template for receiving the screw thread inside a proximal hole or the K-wire inside its own hole (this latter case is shown).

In order to guide this operation it is possible to use a screw guide which is suitably positioned inside the corresponding groove on the fixator and acts both as a spacer and as a stabilizer for the guide wire.

Three screws may be inserted in the tibial plateau, two being parallel, i.e. inserted inside respective holes 16' present in rows parallel to each other, and one converging so as to increase the spongy load-bearing area and at the same time ensure stability both axially and in the other planes. In the distal position three screws are applied, i.e.: two parallel and one converging so as to ensure stability also in a small bone gripping area.

The proximal tip 35 of the screw 3' is screwed a few millimeters into the tibial plateau, the more distal screw 3' is positioned and the respective tip is screwed in a few millimeters.

The distance of the fixator from the bone is set by sliding the fixator 2' along the shank of the screws.

The proximal and distal screws 3' are screwed into the tibial bone until the desired depth is reached, checking also that the stabilization thread 38 engages with the fixator. The other three proximal and distal screws 3' are also inserted depending on the type and position of the fracture.

Essentially, owing to the fixing system according to the present invention, the surgeon manages to stabilize the fixator instantaneously by simply screwing in a pair of screws 3 or 3', adjusting at the same time the alignment and the distance of the body of the fixator 2 or 2' from the fractured bone.

## Claims

1. An integrated orthopedic external fixation system (1), for the treatment of complex or displaced fractures under precarious environmental conditions, of the type comprising an external fixator (2, 2') and a plurality of bone screws (3, 3') for fixing the fixator to a fractured long bone, comprising:
- at least a pair of cylindrical portions (4, 5) interconnected coaxially together by means of interlocking connection means (6, 7) able to adjust the movement relative to each other of these cylindrical portions (4, 5);
- a plurality of through-holes (16, 16') formed transversely with respect to the axis (X) of the body of the fixator (2, 2') and at a predefined distance from the opposite free ends (21, 22) of the fixator (2, 2'), for receiving a respective bone screw (3, 3');
- each bone screw (3, 3') having a smooth cylindrical central section and being slidably engaged in a guided manner inside the corresponding hole (16, 16') of the body of the fixator (2, 2'); and
- a threaded front tip portion, intended to come into screwing engagement with said long bone, in a substantially self-tapping manner;
**characterized in that** said interlocking connection means (6, 7) are able to adjust the movement towards and away from each other of these cylindrical portions (4, 5); and **in that** each bone screw (3, 3') further has
- a threaded rear tail portion intended to come into screwing engagement with the corresponding hole (16, 16') of the fixator body (2, 2'), in a substantially self-tapping manner.

2. The orthopedic system according to claim 1, **characterized in that** each of said cylindrical portions (4, 5) is formed with a relatively soft, but not yielding material, for example a plastic synthetic material or a natural material or a metal alloy.

3. The orthopedic system according to claim 1, **characterized in that** said portions (4, 5) of the cylindrical body of the fixator (2, 2') are coaxially and slidably connected together by means of a male/female joint between the end (7) of one portion (5) and a corresponding end seat (6) of the other portion (4) and **in that** this configuration is locked by means of tightening of a pair of through screw-bolts (8, 9) provided with associated nuts (10, 11); one (9) of said screw-bolts passing through a slot-shaped hole (13) so as to allow a predefined axial sliding movement before tightening of the corresponding nut (11).

4. The orthopedic system according to claim 1, wherein the body of the fixator (2, 2') is cylindrical and made of solid material.

5. The orthopedic system according to claim 1, wherein the body of the fixator (2, 2') is tubular.

6. The orthopedic system according to claim 1, wherein one (5) of said cylindrical portions (4, 5) is longer than the other (4) and two components (4, 5) of the cylindrical body of the fixator (2, 2') are connected together by means of a male/female joint.

7. The orthopedic system according to claim 1, wherein the cylindrical body of the external fixator (2, 2') is provided with a plurality of through-holes (16) distributed uniformly along a substantial section starting from each free end (21, 22) of the cylindrical components (4, 5) and structured to receive a bone screw (3, 3').

8. The orthopedic system according to claim 7, wherein said through-holes (16) are arranged and regularly spaced along at least three parallel lines.

9. The orthopedic system according to claim 8, wherein the center line of said three parallel lines has holes (16) staggered with respect to the holes of the adjacent lines.

10. The orthopedic system according to claim 1, wherein two or more holes (18) with a smaller diameter if compared to the diameter of said through-holes (16) are provided in the vicinity of the free ends (21, 22) of each of the two components (4, 5) of the fixator body (2, 2') for a preliminary stabilization of the fixator using K-wires or Kirschner wires.

11. The orthopedic system according to any of the preceding claims, wherein the tip (25) of the bone screw (3, 3') is self-perforating and self-tapping with an undercut groove (29).

12. The orthopedic system according to any of the preceding claims, wherein said bone screw (3, 3') has a threaded end (30) for bone engagement having a cylindrical shape, obtained by forming an helix on a stem of 5 mm diameter with pitch of 0.75 mm so as to allow a good grip also in a spongy bone.

13. The orthopedic system according to any of the preceding claims, wherein the thread of the bone screw (3, 3') forms a double angle of 30° at the vertex and 150° on the bottom with a thread height of 0.5 mm.

## Patentansprüche

1. Ein integriertes orthopädisches externes Fixationssystem (1) zur Behandlung von komplexen oder verschobenen Frakturen unter prekären Umgebungsbedingungen, des Typs, der einen externen Fixator (2, 2') und eine Vielzahl von Knochenschrauben (3, 3') zur Befestigung des Fixators an einem gebrochenen langen Knochen umfasst, weist auf:
- mindestens ein Paar zylindrischer Abschnitte (4, 5), die koaxial miteinander durch ineinandergreifende Verbindungsmittel (6, 7) verbunden sind, die die Bewegung dieser zylindrischen Abschnitte (4, 5) relativ zueinander einstellen können;
- eine Vielzahl von Durchgangslöchern (16, 16'), die quer zur Achse (X) des Körpers des Fixators (2, 2') und in einem vorbestimmten Abstand von den gegenüberliegenden freien Enden (21, 22) des Fixators (2, 2') zur Aufnahme einer entsprechenden Knochenschraube (3, 3') ausgebildet sind;
- wobei jede Knochenschraube (3, 3') einen glatten zylindrischen Mittelabschnitt hat und gleitend in geführter Weise innerhalb des entsprechenden Lochs (16, 16') des Körpers des Fixators (2, 2') eingreift; und
- einen vorderen Spitzenabschnitt mit Gewinde, der dazu bestimmt ist, in Schraubeneingriff mit dem langen Knochen zu kommen, und zwar im Wesentlichen selbstschneidend;
**dadurch gekennzeichnet, dass** die ineinandergreifenden Verbindungsmittel (6, 7) in der Lage sind, die Bewegung zu und voneinander weg von diesen zylindrischen Abschnitten (4, 5) einzustellen; und dass jede Knochenschraube (3, 3') ferner einen mit Gewinde versehenen hinteren Endabschnitt aufweist, der dazu bestimmt ist, in Schraubverbindung mit dem entsprechenden Loch (16, 16') des Körpers des Fixator (2, 2') in einer im Wesentlichen selbstschneidenden Weise zu kommen.

2. Das orthopädische System nach Anspruch 1, **dadurch gekennzeichnet, dass** jeder der zylindrischen Abschnitte (4, 5) mit einem relativ weichen, aber nicht nachgiebigen Material, zum Beispiel einem Kunststoff, einem Naturmaterial oder einer Metalllegierung, ausgebildet ist.

3. Das orthopädische System nach Anspruch 1, **dadurch gekennzeichnet, dass** die Abschnitte (4, 5) des zylindrischen Körpers des Fixators (2, 2') koaxial und gleitend mittels eines Außen-/Innengelenkes zwischen dem Ende (7) eines Abschnitts (5) und eines entsprechenden Endsitzes (6) des anderen Abschnitts (4) verbunden sind, und dass diese Konfiguration durch Anziehen eines Paares von Durchgangsschrauben (8, 9), die mit zugehörigen Muttern (10, 11) versehen sind, verriegelt wird; wobei einer (9) der Schraubenbolzen durch ein schlitzförmiges Loch (13) verläuft, um eine vorbestimmte axiale Gleitbewegung vor dem Anziehen der entsprechenden Mutter (11) zu ermöglichen.

4. Das orthopädische System nach Anspruch 1, wobei der Körper des Fixators (2, 2') zylindrisch ist und aus Vollmaterial besteht.

5. Das orthopädische System nach Anspruch 1, wobei der Körper des Fixators (2, 2') tubulär ist.

6. Das orthopädische System nach Anspruch 1, wobei einer (5) der zylindrischen Abschnitte (4, 5) länger als der andere (4) ist und zwei Komponenten (4, 5) des zylindrischen Körpers des Fixators (2, 2') über ein Außen- und Innengelenk miteinander verbunden sind.

7. Das orthopädische System nach Anspruch 1, wobei der zylindrische Körper des externen Fixators (2, 2') mit einer Vielzahl von Durchgangslöchern (16) versehen ist, die gleichmäßig entlang eines wesentlichen Teilbereichs verteilt sind, beginnend mit jedem freien Ende (21, 22) der zylindrischen Komponenten (4, 5) und strukturiert sind, um eine Knochenschraube (3, 3') aufzunehmen.

8. Das orthopädische System nach Anspruch 7, wobei die Durchgangslöcher (16) entlang mindestens dreier paralleler Linien angeordnet und entlang dieser regelmäßig beabstandet sind.

9. Das orthopädische System nach Anspruch 8, worin die Mittellinie dieser drei parallelen Linien Löcher (16) aufweist, die in Bezug auf die Löcher der benachbarten Linien versetzt sind.

10. Das orthopädische System nach Anspruch 1, wobei zwei oder mehr Löcher (18) mit einem kleineren Durchmesser im Vergleich zum Durchmesser der Durchgangslöcher (16) in der Nähe der freien Enden (21, 22) jeder der beiden Komponenten (4, 5) des Körpers des Fixators (2, 2') zur Vorstabilisierung des Fixators mit K-Drähten oder Kirschnerdrähten vorgesehen sind.

11. Das orthopädische System nach einem beliebigen der vorhergehenden Ansprüche, wobei die Spitze (25) der Knochenschraube (3, 3') selbstperforierend und selbstschneidend mit einer hinterschnittenen Nut (29) ist.

12. Das orthopädische System nach einem beliebigen der vorhergehenden Ansprüche, wobei die Knochenschraube (3, 3') ein Gewindeende (30) für den Knocheneingriff aufweist, das eine zylindrische Form hat und erhalten ist durch Bilden einer Wendel auf einem Schaft mit einem Durchmesser von 5 mm und einer Steigung von 0,75 mm, um einen guten Griff auch in einem schwammigen Knochen zu ermöglichen.

13. Das orthopädische System nach einem beliebigen der vorhergehenden Ansprüche, wobei der Gewindegang der Knochenschraube (3, 3') einen doppelten Winkel von 30° am Scheitelpunkt und 150° am Boden mit einer Gewindehöhe von 0,5 mm bildet.

## Revendications

1. Système de fixation orthopédique externe intégré (1), pour le traitement de fractures complexes ou déplacées dans des conditions d'environnement précaire, du type comprenant un élément de fixation externe (2, 2') et une pluralité de vis à os (3, 3') pour fixer l'élément de fixation à un os long fracturé, comprenant :
- au moins une paire de parties cylindriques (4, 5) raccordées entre elles coaxialement au moyen de moyens de raccordement mutuel (6, 7) capables d'ajuster le mouvement l'une par rapport à l'autre de ces parties cylindriques (4, 5) ;
- une pluralité de trous traversants (16, 16') formés transversalement par rapport à l'axe (X) du corps de l'élément de fixation (2, 2') et à une distance prédéfinie des extrémités libres opposées (21, 22) de l'élément de fixation (2, 2'), pour recevoir une vis à os respective (3, 3') ;
- chaque vis à os (3, 3') ayant une section centrale cylindrique lisse et étant en prise coulissante d'une manière guidée à l'intérieur du trou correspondant (16, 16') du corps de l'élément de fixation (2, 2') ; et
- une partie de pointe avant filetée, conçue pour venir en prise par vissage avec ledit os long, sensiblement à la manière d'un auto-taraudage ;
**caractérisé en ce que** lesdits moyens de raccordement mutuel (6, 7) sont capables d'ajuster le mouvement en direction des et depuis chacune des deux parties cylindriques (4, 5) ; et **en ce que** chaque vis à os (3, 3') présente en outre
- une partie de queue arrière filetée conçue pour venir en prise par vissage avec le trou correspondant (16, 16') du corps d'élément de fixation (2, 2'), sensiblement à la manière d'un auto-taraudage.

2. Système orthopédique selon la revendication 1, **caractérisé en ce que** chacune desdites parties cylindriques (4, 5) est formée avec un matériau relativement mou, mais pas élastique, par exemple un matériau plastique synthétique ou un matériau naturel ou un alliage métallique.

3. Système orthopédique selon la revendication 1, **caractérisé en ce que** lesdites parties (4, 5) du corps cylindrique de l'élément de fixation (2, 2') sont raccordées de façon coaxiale et coulissante l'une à l'autre au moyen d'un joint mâle/femelle entre l'extrémité (7) d'une partie (5) et un siège d'extrémité correspondant (6) de l'autre partie (4) et **en ce que** cette configuration est verrouillée au moyen du serrage d'une paire de boulons à vis traversants (8, 9) dotés d'écrous associés (10, 11) ; l'un (9) desdits boulons à vis passant à travers un trou en forme de fente (13) de manière à permettre un mouvement de coulissement axial prédéfini avant le serrage de l'écrou correspondant (11).

4. Système orthopédique selon la revendication 1, dans lequel le corps de l'élément de fixation (2, 2') est cylindrique et composé d'un matériau solide.

5. Système orthopédique selon la revendication 1, dans lequel le corps de l'élément de fixation (2, 2') est tubulaire.

6. Système orthopédique selon la revendication 1, dans lequel l'une (5) desdites parties cylindriques (4, 5) est plus longue que l'autre (4) et deux composants (4, 5) du corps cylindrique de l'élément de fixation (2, 2') sont raccordés l'un à l'autre au moyen d'un joint mâle/femelle.

7. Système orthopédique selon la revendication 1, dans lequel le corps cylindrique de l'élément de fixation externe (2, 2') est doté d'une pluralité de trous traversants (16) répartis uniformément le long d'une section substantielle à partir de chaque extrémité libre (21, 22) des composants cylindriques (4, 5) et structurés pour recevoir une vis à os (3, 3').

8. Système orthopédique selon la revendication 7, dans lequel lesdits trous traversants (16) sont agencés et régulièrement espacés le long d'au moins trois lignes parallèles.

9. Système orthopédique selon la revendication 8, dans lequel la ligne centrale desdites trois lignes parallèles présente des trous (16) décalés par rapport aux trous des lignes adjacentes.

10. Système orthopédique selon la revendication 1, dans lequel deux ou plus de deux trous (18) présentant un diamètre inférieur par rapport au diamètre desdits trous traversants (16) sont disposés au voisinage des extrémités libres (21, 22) de chacun des deux composants (4, 5) du corps d'élément de fixation (2, 2') pour une stabilisation préliminaire de l'élément de fixation au moyen de broches K ou broches de Kirschner.

11. Système orthopédique selon l'une quelconque des revendications précédentes, dans lequel la pointe (25) de ladite vis à os (3, 3') est à auto-perforation et auto-taraudage avec une rainure en contre-dépouille (29).

12. Système orthopédique selon l'une quelconque des revendications précédentes, dans lequel ladite vis à os (3, 3') présente une extrémité filetée (30) pour une entrée en prise d'os ayant une forme cylindrique, obtenue par la formation d'une hélice sur une tige de 5 mm de diamètre avec un pas de 0,75 mm de manière à permettre une bonne prise également dans un os spongieux.

13. Système orthopédique selon l'une quelconque des revendications précédentes, dans lequel le filet de la vis à os (3, 3') forme un double angle de 30° au niveau du sommet et de 150° au niveau du fond avec une hauteur de filet de 0,5 mm.
